# EUROPEAN PATENT SPECIFICATION

(11) **EP 3 429 627 B1**
(45) Date of publication and mention of the grant of the patent: **21.08.2024**
(21) Application number: 17715274.1
(22) Date of filing: 10.03.2017
(51) Int. Cl.: A61K 47/32, A61K 9/107, A61L 24/00, A61K 49/04, A61K 9/16, A61P 35/00, A61P 41/00

(54) **EMULSION COMPRISING PARTICLES**
EMULSION ENTHALTEND PARTIKEL
ÉMULSION COMPRENANT DES PARTICULES

(30) Priority: 14.03.2016 US 201662307884 P
(43) Date of publication of application: 23.01.2019
(73) Proprietor: Boston Scientific Medical Device Limited, Galway (IE)
(72) Inventor: DREHER, Matthew, R, West Conshohocken, PA 19428 (US); LEWIS, Andrew, Lennard, Camberley Surrey GU15 3YL (GB); WILLIS, Sean, Leo, Camberley Surrey GU15 3YL (GB); CHUNG, Shui Ting, Farnham Surrey GU8 8QL (GB); CAINE, Marcus Gordon, Farnham Surrey GU8 8QL (GB)
(74) Representative: Peterreins Schley
(86) International application number: PCT/IB2017/051416
(87) International publication number: WO 2017/158482

(56) References cited:
- EP-A1- 2 365 009
- WO-A2-2008/054205
- DATABASE BIOSIS [online] BIOSCIENCES INFORMATION SERVICE, PHILADELPHIA, PA, US; February 2006 (2006-02-01), DUDECK OLIVER ET AL: "Intrinsically radiopaque iodine-containing polyvinyl alcohol as a liquid embolic agent: evaluation in experimental wide-necked aneurysms", Database accession no. PREV200600250209
- DUDECK OLIVER ET AL: "Intrinsically radiopaque iodine-containing polyvinyl alcohol as a liquid embolic agent: evaluation in experimental wide-necked aneurysms", JOURNAL OF NEUROSURGERY, vol. 104, no. 2, February 2006 (2006-02-01), pages 290 - 297, ISSN: 0022-3085(print)
- OLIVER DUDECK: "Intrinsically radiopaque iodine-containing polyvinyl alcohol as a liquid embolic agent: evaluation in experimental wide-necked aneurysms", JOURNAL OF NEUROSURGERY, vol. 104, no. 2, 1 January 2006 (2006-01-01), pages 290 - 297, XP055371047

## Description

The present invention relates to the field of therapeutic embolisation and particularly relates to materials and methods for carrying out transcatheter arterial chemoembolisation (TACE).

Therapeutic embolisation is a minimally invasive technique in which blood flow to tissue is restricted by blocking vessels supplying the tissue, resulting in local necrosis; both liquid and solid embolic agents have been used. In one approach, the effect is achieved by the transcatheter delivery of an oily emulsion, which may additionally comprise antineoplastic drugs. The oil component is typically a composition of iodised ethyl-esters of the fatty acids of poppy seed oil (Lipiodol^{®} Ultra Fluide - Guerbet S. A., France). The aqueous component typically comprises an iodinated contrast agent to improve visualisation during delivery. This approach has been useful in the treatment of hepatocellular carcinoma (HCC) for example and is known as conventional TACE (cTACE).

The emulsion is introduced into the branch of the hepatic artery feeding the carcinoma and typically embolises not only the tumour-feeding hepatic arterioles but also portal vein venules, by crossing the peribiliary plexus between the two systems feeding the tumour or across the sinusoids. However, emulsions are weak embolic agents and are rapidly washed out, liberating cytotoxic drugs into the general circulation. Use of particulate "plugs" following delivery (for example protein foam particles, or permanent microspherical embolics - see for example Geschwind et al Cardiovasc. Intervent. Radiol. (2003) 26:111-117) is helpful in prolonging the embolic effect, but does not ultimately prevent dispersion of the emulsion. Further, following cTACE, the oil and drug are not co-localised (Gaba R., et al J.V.I.R. 2012; 23(2); 265-273).

The emulsion must be sufficiently stable for convenient preparation and delivery. It should also remain stable during delivery through the small catheters required for the procedure and remain cohesive as it travels down the blood vesssel. Further, should re-emulsification be necessary, the re-prepared emulsion should have similar properties to the original. However variations in preparation and materials can lead to widely varying properties, such as stability.

Embolic particles, of various, materials, are available; polymer microspheres, typically with diameters in the range 40 - 1200 uM, are one such material. In some cases they may be adapted for TACE by incorporating a chemotherapeutic agent, which is released over a period of hours or days. This approach has also been used in the treatment of HCC although due to their size, embolic particles do not typically pass into the hepatic portal venules and so a proportion of the tumour circulation is not accessible. WO2004/071495 discloses one such agent, prepared from a poly vinylalcohol co-polymer.

Despite their drawbacks, emulsion preparations for cTACE remain popular. However, there remains a need for preparations for use in cTACE, that provide predictable pharmacokinetics, with reduced whole body exposure, that provide a good degree of embolisation, that deliver drug to the tissue over an extended period, that are simple and rapid to prepare at the point of use, that requires a minimum number of components, that provide for the potential of portal vein embolisation, that provide radiographic feedback and are reliably stable for sufficient time, both before and during delivery.

WO2015/036626 provides emulsion formulations comprising hydrophilic particles and Lipiodol^{®} however these can suffer stability issues and re-emulsification leads to progressively shorter stability times.

EP 2 365 009 A1 discloses radadiopaque, non-biodegradable, water-insoluble iodinated benzyl ethers of poly(vinyl alcohol).

The article in JOURNAL OF NEUROSURGERY, vol. 104, no. 2, 1 January 2006, pages 290-297, discloses intrinsically radiopaque iodine-containing polyvinyl alcohol as a liquid embolic agent.

WO 2008/054205 A2 discloses embolic materials comprising spherical, homogeneous and substantially non-porous radiopaque polymer particles based on at least one hydrophilic monomer and at least one radiopaque monomer.

The present inventors have identified that an emulsion formulation with improved characteristics for use in TACE can be easily prepared prior to delivery, by incorporating embolic particles into the composition. Particularly, the inventors have identified that by incorporating particles that comprise an iodinated polymer the characteristics of a emulsion can be altered to increase stability and usability. The preparation can be made easily at the point of use, requires no additional special equipment to make and has an improved stability over previously disclosed emulsion formulations. The preparation has the potential to provide embolisation of both the arterial and portal venous supply to the tumour, and to reduce exposure of the general circulation to incorporated drug and to provide more predictable pharmacokinetics and improved local delivery. Particle hydrophobicity is increased by incorporation of hydrophobic moieties into the polymer, particularly useful are iodinated aromatic species that not only increase hydrophobicity, notably surface hydrophobicity, but also impart inherent radiopacity to the particles.

### Statement of invention

Accordingly the invention provides an emulsion composition comprising a continuous phase, a discontinuous phase and a plurality of particles, the discontinuous phase being aqueous and the continuous phase comprising an oil; wherein the particles comprise a hydrophilic polymer to which iodine is covalently bound.

### Particle Characteristics

### Shape

Although irregularly shaped particulate material may be used in the present invention, spherically shaped particles are generally preferred, because their spherical form leads to good flow properties for more distal distribution and ease of handling. The type of particle known in the art as a microparticle, or preferably a microsphere is particularly preferred.

### Size

Any size of particle or microsphere that is suitable for embolisation therapy can be used, but in general they will not exceed 2000um in diameter. Since it is preferred that particles do not pass through the capillary bed to the venous system beyond the tumour, particles of at least 15um are preferred. Particles are generally provided in a range of sizes, for example particles of 15 to 1200 microns may be used; preparations typically provide particles in size sub-ranges to suit the planned treatment, for example 100-300, 300-500, 500-700 or 700-900 um. Smaller particles tend to pass deeper into the vascular bed and so for some purposes, particles in ranges such as 15-35, 30-60, 40-90 or 70-150 microns, for example, are preferred. Where particles are referred to by size range, this means that the range encompasses at least 80% of particles in the preparation, preferably 90%, unless otherwise stated. Size refers to fully hydrated particles in normal saline (1mM sodium phosphate pH7.2-7.4, 0.9%NaCl).

### Porosity

Particles having internal spaces such as pores or voids are also contemplated. Pores may be interconnected or not, but are open to the exterior of the particle. Voids are internal spaces not connected to the exterior.

### Dried

The particles may be provided in a dried form or in hydrated form. Dried particles are typically provided lyophilised and stored under vacuum (i.e. at a pressure of less than 0.01bar). Where hydrated particles are referred to herein, this refers to particles fully hydrated in normal saline (1mM sodium phosphate pH7.2-7.4, 0.9%NaCl) unless otherwise stated. All measurements are at lab temperature, between 18 and 22°C, unless otherwise stated. In one preferred embodiment, particles are dried in the presence of one or more lyoprotectants. This helps to preserve the structure of the particle when dried. Suitable lyoprotectants include pharmaceutically acceptable water soluble polyhydroxy compounds such as polyalcohols and mono, di and polysaccharides. For example sucrose, glucose, dextrose and trehalose may be used; mannitol has provided good results. Lyoprotectants are particularly useful in cases where removal of structural water may prejudice the structure of the particle, for example in the case of particles prepared from hydrogels. Particles treated in this way have improved drug loading characteristics.

### Polymers

The particles of the invention comprise a polymer. Specifically, the particles comprise a hydrophilic polymer to which iodine is covalently bound. Many types of polymer may be used for the preparation of the particles and they may be biodegradable or not biodegradable. In a preferred embodiment, the polymer is water swellable, but not water soluble. Cross linked polymers are particularly preferred. Polymers include natural polymers such as polysaccharide (e.g. chitosan or alginate) or proteins (such as albumen or gelatin) or synthetic polymers.

Synthetic polymers include polyesters such as polylactides, polyglycolides and co-polymers of these, such as polylactide-co-glycolides and poly(D, L-lactide-co-PEG), polyorthoesters and poly(ester amides); acrylates, (such as methacrylates) acrylamides (such as methacrylamides), polyureas, polyurethanes, polyolefins, polyvinylhalides, polyvinylidenehalides, polyvinylethers, polyvinylaromatics, polyvinyl esters, polyacrylonitriles, alkyd resins, polysiloxanes, epoxy resins, vinylalcohol polymers, polyphosphazenes, polyphosphoesters, polyphosphoester polyhydroxyalkanoates, polyanhydrides, polyamino acids, polyoxymethylenes, and polyimides; polycaprolactones, polyvalerolactones, polyanhydrides, polyethylene glycols, polyethylene oxides, including acyl polyethylene oxides and their co-polymers, pyrrolidones and vinyl pyrrolidones as well as co-polymers of the above such as those with monomers based on acrylic acid, acrylamides, and acrylates.

According to the invention, the particles (microspheres) of the invention comprise covalently bound iodine i.e., the particle comprises a polymer (as described above) to which iodine is covalently bound.

### Charge

The polymers of the particle preferably carry an over all charge at a pH in the range 6-8, which assists in binding charged drugs. The particles may be cationically charged, for example by having free quaternary ammonium groups or anionically charged, for example by having free carboxylate or sulphonate groups. Preferably they are anionically charged at a pH between 6-8. Conveniently the charge can be introduced into the polymer by incorporating a charged co-monomer

### Hydrogels

Hydrogel polymers are preferred, because they allow easy passage of loaded drugs to all parts of the polymer of the particle. Polymers that are water swellable, but not water soluble are preferred. Hydrogels comprising covalently bound iodine and comprising greater than 30% water (w/w) are preferred. Such hydrogels comprising at least 40% water have provided good results. Hydrogels may have up to 99% water w/w, but typically will have up to 80 or 90% water w/w. Within this range sub ranges such as 54-82%, 50-85%, 54-82%, 50-75% and 60-55% are preferred.

Preferred polymers are polymers and co-polymers of polyvinyl alcohol (PVA) particularly where the polymer is crosslinked, preferably either physically or covalently. Particularly preferred are co-polymers such as those with acrylamides, methacrylamides, acrylates or methacrylates. Examples of such polymers include PVA co-sodium acrylate (eg Ex.1 of WO2006/119968) or PVA modified with N-acryloyl-aminoacetaldehyde dimethylacetal (NAAADA) to form a PVA macromer, and crosslinked with 2-acrylamido-2-methylpropanesulfonic acid (AMPS) as described in WO04071495, example 1 (PVA-AMPS).

Also preferred are polymers and co-polymers of polyethylene glycol, particularly polymers of PEG macromers such as those of PEG-acrylamides (eg PEG-diacrylamide), or PEG-methacrylate. These include those polymers and co-polymers of polyethylene glycol, cross linked with charged monomers, such as 3-sulphopropyl acrylate or 2-acrylamido-2-methylpropanesulfonic acid (see for example WO 2015/070094).

Also preferred are acrylic polymers, such as those of acrylates and methacrylates such as polymethacrylic acid (see for example Thanoo et al., J. Appl. P. Sci., 1990 (38), 1153-1161 and US4,622,367). Such polymers may be prepared from unhydrolyzed precursors such as methyl acrylate (MA), methyl methacrylate (MMA), ethylmethacrylate (EMA), hexamethylmethacrylate (HMMA) or hydroxyethyl methacrylate (HEMA) and subsequently hydrolysed. Suitable cross linkers include glycol-based materials such as ethylene glycol dimethacrylate (EGDMA), diethylene glycol dimethacrylate (DEGDMA) or particulalry, triethylene glycol dimethacrylate (TEGDMA). Particularly preferred are particles comprising methacrylic acid crosslinked with TEGDMA, which may be coated with polyphosphazine coating, such as poly(trifluoroethoxy)phosphazine (as described in WO2006/046155).

### Iodination

The unit of iodine content in the particles described herein is milligrams of iodine per millilitre of beads (mg I/ml). This refers to the iodine content in the beads as packed bead volume, fully hydrated in saline. That is to say volume measured as if by measuring cylinder.

In a preferred embodiment, the particles (microspheres) of the invention comprise covalently bound iodine i.e., the particle comprises a polymer (as described above) to which iodine is covalently bound. The coupling of iodinated moieties to the polymer increases the hydrophobic nature of the polymer, so that, for example, although in one embodiment the polymer of the particles is hydrophilic and may be water swellable, but water insoluble, the nature of the polymer is altered by the coupling of the iodine or iodinated moiety to increase the hydrophobic nature of the polymer. Preferably the iodine is bound substantially throughout the particle.

Preferably the polymers comprise at least 20mg I/ml of beads. Preferably at least 30mg I/ml and more preferably at least 60 at least 100mg, at least 130 or at least 150 I/ml. The upper level of iodine is typically governed by the ability to visualise the bead under X-ray and other factors such as effect on ability of the particle to bind and release drug, density of the bead and so on, and would be established by the skilled person in accordance with requirements. Particles with an iodine content of 155mg I/ml have a radiopacity of 6769 HU and are considered to provide a good level of radiopacity, to be adequately visible by fluorography, to provide good stability of emulsions and load and release drugs such as doxorubicin well. Nevertheless, the upper limit of iodine content may be up to 200 or 250mg I/ml. ranges of 30 to 200 mg I/ml are considered to be useful, for example 30-175, 30-160, 50-200, 60-200, 100-175, 100-200 and 125 -160mg I/ ml. Examples demonstrate particles with an iodine content of 33-155mg I/ml.

The iodine is preferably bound to the polymer through an aromatic group, such as a phenyl group, which is itself preferably covalently attached to the polymer. Thus 1, 2, 3, or 4 iodines may be bound to the polymer through such a group, for example as a mono, di, tri or tetraiodophenyl group. This allows convenient attachment of a high density of iodine.

Several approaches to coupling such groups to polymers are known. For example, the iodine may be introduced during the synthesis of the polymer, for example by forming the polymer from an iodinated monomer, or co-monomer. Iodinated monomers such as acrylates, methacrylates and acrylamides have been used. 2-(2'-iodobenzoyl)ethyl methacrylate or 2-(2',3',5'-triiodobenzoyl)ethyl methacrylate which may be coupled with other ethylenically unsaturated monomers are described by Benzina, et al (Biomat. 15(14) 1122-1128 (1994) and J. Biomed. Mater. Res. 32(3), 459-466 (1996)).

This approach is also used to prepare iodinated cross linked Tris hydroxymethyl methylacrylamides by incorporating acrylamido-3-propionamido )-3-triiodo-2,4,6-benzoic acid (US5648100).

An alternative method involves coupling iodinated groups to preformed particles such as microspheres (suitably aromatic groups, e.g iodinated phenyl groups). This can be achieved, for example by coupling reactive groups, such as acid chlorides on the iodinated group, with suitable functional groups on the polymer (e.g. with the hydroxyl groups of 2-hydroxy acrylates (for example 2-hydroxyethyl methacrylate groups in HEMA particles see US4622367). Alternatively alcohol (eg triiodobenzyl alcohol) acid (eg triiodobenzoic acid), amine (eg iodobenzylamine) or other suitable derivatives of the iodinated aromatic group may be coupled to the polymer using carbodiimide or carbodiimidazole coupling (see for example WO 2014/152488).

In a preferred embodiment, polymer particles having a polymer backbone comprising 1,2 or 1,3, diols, (e.g. PVA) are coupled with an iodinated aromatic aldehyde, such as 2,3,5 triiodobenzaldehyde, such that the (PVA) backbone comprises iodinated aromatic groups (such as triiodophenyl groups) covalently coupled through a cyclic acetal such as described in WO2015/033092 (formula I):

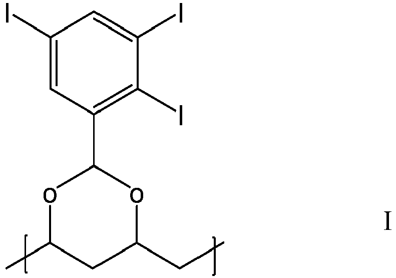

In a preferred embodiment therefore, the particles comprise polymers or co-polymers of PVA. The polymer is cross-linked, preferably either physically or covalently and comprises at least 20mg I /ml preferably up to 250mg I/ml, wherein the iodine is covalently coupled to the polymer, preferably through an aromatic group, which itself is covalently coupled to the polymer; the aromatic group bearing 1,2,3 or 4 iodines. The approach is particularly preferred when the PVA polymer is a PVA-co-sodium acrylate or PVA-AMPS polymer (WO04071495).

### Oils

Oils are non-mineral (ie organic) oils, particularly plant derived oils, and should preferably be pharmaceutically acceptable by the injection route.

Whilst the present invention is described in terms of the use of Lipiodol , which provides particular advantages, (such as radiopacity, due to incorporated iodine), other oils may be used. Oils comprising water-insoluble lipids, particularly mono, di, or triglycerides, or their free fatty acids, hydrogenated products of them or esters (e.g. methy ethyl and propyl, preferably ethyl esters) thereof, and/or halogenated (e.g iodinated or brominated) products of these are preferred, due to their potential to be metabolised easily. For example poppy seed oil, castor oil, corn oil, cottonseed oil, olive oil, peanut oil, peppermint oil, safflower oil, sesame oil, soybean oils and their free fatty acids (such as linoleic, oleic, palmitic and stearic acids), hydrogenated products of these or esters (e.g. methy ethyl and propyl, preferably ethyl esters) thereof and/or halogenated (e.g iodinated or brominated) products of these. Oils comprising ethyl esters of stearate and particularly iodinated products of these are preferred such as ethyl monoiodostearate and ethyldiiodostearate.

### Composition

### Oil:aqueous ratio

The preferred emulsion is of the water-in-oil type; with this structure, the aqueous phase, within the oil, is protected from the blood when it exits the catheter. This prevents water droplets being immediately dispersed in the blood leading to dispersal of any drug present in the emulsion into the circulation. The ratio of oil (Lipiodol) phase to aqueous phase in the emulsion should be greater than 1:1 vol/vol Lipiodol: aqueous phase, i.e. the proportion of Lipiodol in the emulsion should exceed the proportion of aqueous phase. Preferably the ratio is at least 1.1:1, or at least 1.5:1, for example between 1.1:1 and 10:1 or 5:1. In particular the ranges 1.5:1 to 10:1, or 1.5:1 to 6:1 or 5:1.

### Particle:Lipiodol ratios

In general the quantity of particles to be to be used in the preparation will depend on the extent of embolisation required and the quantities of particles required to reach blood flow stasis. The minimum quantity for any emulsion preparation will be that which stabilises the emulsion, and will vary depending on the constituents of the emulsion. Typically the ratio of particles to Lipiodol in the composition will be between 1:100 and 1:1, preferably 1:20 or 1:40 to 1:1 v/v and more preferably between 1:10 or 1:5 and 1:1.

Volume refers to packed volume (such as may be measured using a measuring cylinder) of fully hydrated particles in normal saline (1mM sodium phosphate pH7.2-7.4, 0.9%NaCl) before the particles are dried (see Example 1).

### Stability

The emulsions of the invention demonstrate improved stability and are typically stable for at least 10 minutes, preferably at least 30 min, more preferably at least 60 mins, more preferably at least 80 mins or 90mins. Where stability is measured according to Example 3a and stability is defined as the time taken for 10% of the emulsion volume to separate.

The emulsions of the invention also remain cohesive in flow. Emulsions form stable oil droplets in the static flow test described in example 3c and tend to remain stable at bifurcation points in the continuous flow test of example 3d.

### Aqueous phase

The aqueous phase may be for example, water or saline (e.g 1mM sodium phosphate pH7.2-7.4, 0.9%NaCl) and may comprise additional components such as drugs or contrast agents.

### Contrast agents

Contrast agents may be any component that allows the detection of the aqueous phase (and hence the emulsion) by an imaging modality (such as X-ray, magnetic resonance, ultrasound, positron emission contrast agents. Such agents include iodine containing contrast media for X-ray based systems such as fluoroscopy or projectional radiography, gadolinium, iron, platinum or manganese containing contrast media for MRI; gas bubble preparations, such as gas-containing microballoons for ultrasound or 18F containing compounds, or 64Cu or 68Ga for positron emission modalities. Typically the contrast agent will be an X-ray contrast agent such as an iodine containing compound. Particularly a non-ionic iodine containing contrast agent such as iopamidol, iohexol, iodixanol or iopromide.

### Drug

The present composition is particularly advantageous when it comprises one or more pharmaceutical actives, which may be present in the particles, the oil and/or aqueous phase. The presence of oil, water and particle phases provides the opportunity to incorporate a different type of drug into each, for example the invention contemplates the provision of emulsions in which the oil phase comprises a hydrophobic drug (which may be in solid form or dissolved in the oil), whilst the aqueous phase comprises a hydrophilic drug (which may be in solid form or dissolved in the aqueous phase). The invention also contemplates the provision of different hydrophilic drugs in the particle and aqueous phase.

Preferably the particle comprises a pharmaceutical active, which is preferably releasably bound to the particle by ionic interaction.

Antineoplastic drugs are preferred. Particularly camptothecins (such as irinotecan and topotecan), anthracyclines (such as doxorubicin), platinum containing drugs (such as spiroplatin, cisplatin, oxaliplatin, miriplatin or carboplatin), antimetabolites (such as thymidyate synthase inhibitors such as 5-FU), Kinase inhibitors (such as inhibitors of VEGFR and EGFR e.g. sunitinib, sorafenib, erlotinib, gefitinib and vandetanib), mitotic poisons (such as the Taxanes e.g paclitaxel or docetaxel; or Vinca alkaloids, e.g. vinblastine and vincristin and synthetic versions such as vinorelbine), aromatase inhibitors such as anastrozole), inhibitors of 17 α-hydroxylase/C17,20 lyase (CYP17A1), (e.g. abiraterone or its acetate), antifolates (such as methotrexate), hormone receptor antagonists (such as tamoxifen and degarelix) or agonists (such as buserelin), alkylating agents (such as chlorambucil, busulfan, streptozotocin, lomustine and cyclophosphamide), retinoid activators (such as bexarotene),

Where particles are charged, it is preferred that the active carries the opposite charge under the same conditions, in order to promote loading of the drug. Where the active is not charged this can be achieved by providing the active in a salt form. In a preferred combination the particle comprises a polymer which is anionically charged at pH6-8 as described above, and bound to the polymer in releaseable form a cationically charged drug. Particularly preferred are drugs suitable for use in TACE.

### Contemplated drugs include:

Actinomycin D, abiraterone or its acetate, aldesleukin, alitretinoin, allopurinol, altretamine, amifostine, aminoglutehimide, amphotericin B, amsacrine, anastrozole, ansamitocin, arabinosyl adenine, bendamustine, benzamide, bexarotene, bleomycin, 3-bromopyruvate, buserelin, busulfan, calusterone, capecitabine, carboplatin, chlorambucil, carboplatin cisplatin, miriplatin, spiroplatin, carzelesin, carmustine, celecoxib, chlorambucil, cladribine, cyclophosphamide, cytarabine, fludarabine, dacarbazine, doxorubicin, daunorubicin, epirubicin, idarubicin, denileukin diftitox, dexamethasone, dromostanolone, degarelix, erlotinib, gefitinib, imatinib, laptinib, sunitinib, sorafenib, estramustine, etoposide, exemestane, filgrastim and PEGylated derivatives, 5-FU, floxuridine, flutamide, fulvestrant, demcitabine, gemcitabine, goserelin acetate, hydroxyurea, idarubicin, ifosfamide, interferon , irinotecan, topotecan lanreotide, lenalidomide, letrozole, leucovorin, leuprolide, leuprorelin, lomustine, meciorthamine, megestrol, melphalan, mercaptopurine, mercaptopolylysine, methotrexate, pemetrexed, raltitrexed, methoxsalen, mithramycin, mitomycin, mitotane, mitoxantrone, nandrolone phenpropionate, octreotide, oprelvekin, oxaliplatin, paclitaxel, pamidronate sodium, pentostatin, pipobroman, plicamycin, porfimer sodium, procarbazine, quinacrine, raltitrexed, streptozotocin, tamoxifen, tegafur-uracil, temozolomide, teniposide, testolactone, tioguanine, thioTEPA, topotecan, toremifene, treosulfan, tretinoin, trilostane triptorelin, valrubicin, vandetanib, vinblastine, vincristine, vindesine, vinorelbine, zoledronate.

Doxorubicin, idarubicin, mitomycin, mitoxantrone, epirubicin, daunorubicin, irinotecan, topotecan, sunitinib, vandetanib, miriplatin and sorafenib are particularly preferred.

### The emulsion

The emulsions of the invention comprise particles which tend to congregate in the aqueous phase. Particles in the aqueous phase are protected within the emulsion on exiting the catheter. It is preferred therefore that the majority of particles are associated with the aqueous phase.

### Hydrophobicity

Without wishing to be bound by any theory, the inventors have identified that by iodinating the particles the hydrophilic nature of the polymer is altered, to increase the hydrophobic character of the polymer.

A further aspect of the invention therefore provides an emulsion composition comprising a continuous phase, a discontinuous phase and a plurality of particles, the discontinuous phase being aqueous and the continuous phase comprising an oil; wherein the particles are sufficiently hydrophobic such that an emulsion prepared according to example 2 herein, using a lipiodol:aqueous phase ratio of 2:1 and in which the aqueous phase contains no contrast agent, is stable for at least 10, preferably at least 30, preferably at least 60, more preferably at least 80 and most preferably at least 90 minutes at between 18 and 22°C.

An approximation of the relative hydrophilicity/hydrophobicity of the particles can be obtained using Contact Atomic Force Spectroscopy (CAFS) using force-distance-amplitude protocols and a silicon probe tip. This provides a measure of the silicon tip interaction with the surface of the particle analysed in air as a dry powder.

The inventors have determined that, when measured according to the protocol laid out in Example 4a, particles having a cantilever deflection (measured in volts) of less than that of the non-iodinated particle were effective at stabilising the emulsion.

Particularly, particles having a cantilever deflection (CD) less than that of DC Bead (70-150)um when lyophilised and measured with a silicon probe were effective. Particles having a CD of less than or equal to 0.2V are preferred, particularly those having a CD of less than or equal to 0.15 or 0.1V.

Alternatively those particles having a CD which is at least 0.5, preferably at least 0.6, and more preferably at least 0.7V less than that of DC Bead are preferred.

Alternatively those particles having a CD less than or equal to 0.25 times the CD of DC Bead, preferably less than or equal to 0.2 and more preferably less than or equal to 0.15 or 0.125x the CD of DC Bead are preferred.

### Statement

Accordingly a further embodiment provides an emulsion composition comprising a continuous phase, a discontinuous phase and a plurality of particles, the discontinuous phase being aqueous and the continuous phase comprising an oil; wherein the particles when measured according to the protocol laid out in Example 4a, have a cantilever deflection (measured in volts) of less than that of DC Bead.

### Process

A further aspect of the invention provides a process for preparing an emulsion as described above comprising:
a. providing a continuous phase comprising an oil as described herein;
b. providing an aqueous phase as described herein
c. providing a plurality of particles comprising a polymer to which iodine is covalently bound as described herein; and
combining them to provide an emulsion.

The preferred features of the composition and the components thereof are described above. Preferably the particles are provided lyophilised. Preferably the particles are combined with the oil phase before adding the aqueous phase. Preferably the aqueous phase comprises either a contrast agent as described herein, or a pharmaceutical active as described herein, or both. during the formation of the emulsion, the drug is rapidly taken up into the particle, especially in the case where the polymer is charged and the drug carries the opposite charge, as described above. This loading process is accelerated when using dried particles, so when drug is used the particles are preferably lyophilised. Preferably, when the particles are provided lyophilised, the aqueous phase or the oil phase, are combined with the particles whilst they are still under vacuum. Conveniently the emulsion is formed by passing the components between two syringes.

It is preferable to mix the components by passing them back and forth through an orifice that provides sufficient turbulence to form the emulsion, conveniently this is achieved by passing the components back and forth between two syringes, through a connector such as a stop-cock. The resulting emulsion is stable for at least 10 minutes, preferably at least 30 min, more preferably at least 60 mins, more preferably at least 80 mins or 90mins. Where stability is measured according to Example 3a and stability is defined as the time taken for 10% of the emulsion volume to separate.

A further aspect of the invention is an emulsion composition according to any aspect of the invention preparable (or when prepared) by a process as described above

### Methods of treatment

A further aspect of the invention provides a method of embolotherapy in a patient having a tumour, and in need of such embolotherapy, comprising providing an embolic emulsion composition as described herein and delivering the composition to blood vessels of the tumour, delivery is typically such as to reduce the blood flow to the tumour.

The tumour is typically a hypervascular tumour, such as a hepatocellular carcinoma. The composition is preferably delivered in an amount sufficient to reduce the blood flow to the tumour, particularly to flow stasis or near stasis in the vessels embolised. The composition is typically delivered by catheter into a vessel supplying the tumour, such that blood flow carries the composition into the tumour. At least on leaving the catheter, particles remain associated with the emulsion phase and preferably remain within droplets of water in oil emulsion

### Second medical use

A further aspect of the invention provides a pharmaceutical active (as described herein) for use in the treatment of a tumour by embolotheraphy, wherein the pharmaceutical active is delivered, in an emulsion composition according to the invention, as described herein.

The pharmaceutical active is preferably an antineoplastic drug, and is preferably selected from doxorubicin, idarubicin, mitomycin, mitoxantrone, epirubicin, daunorubicin, irinotecan, topotecan, sunitinib, vandetanib, miriplatin and sorafenib.

In one particularly preferred embodiment, the emulsion composition comprises a continuous phase, a discontinuous phase and a plurality of particles, the discontinuous phase being aqueous and the continuous phase comprising an oil; wherein either:
a) the particle comprises a polymer to which iodine is covalently bound,
b) the particles are sufficiently hydrophobic such that an emulsion prepared according to example 2 herein, using a lipiodol:aqueous phase ratio of 2:1 and in which the aqueous phase contains no contrast agent, is stable for at least 10, preferably at least 30, preferably at least 60, more preferably at least 80 and most preferably at least 90 minutes at between 18 and 22°C; or
c) the particles, when measured according to the protocol laid out in Example 4a, have a cantilever deflection (measured in volts) of less than that of DC Bead.

The preferred features of the invention are as described above.

### Kits

The invention also provides kits for preparing the emulsion of the invention and so the a further aspect of the invention provides a kit for preparing an emulsion comprising and oil and a plurality of particles, the particles comprising iodine which is covalently bound to the particle, the kit comprising a plurality of particles, as described herein, which are preferably provided lyophilised, and an oil as described herein. The kit may also provide aqueous phase, which is preferably either water for injection or saline and may additionally comprise a drug, which may be any of those described herein but is preferably selected from doxorubicin, idarubicin, mitomycin, mitoxantrone, epirubicin, daunorubicin, irinotecan, topotecan, sunitinib, vandetanib, miriplatin and sorafenib,

The invention will now be described further using the following non limiting examples and figures in which:

### Figures

***Figure 1*** illustrates bead density and moisture content for beads of varying iodine content.
***Figure 2*** illustrates emulsion stability and bead sedimentation for emulsions prepared with beads of varying iodine content using an emulsion of 2:1 oil to aqueous.
***Figure 3*** compares photomicrographs of emulsions prepared using non iodinated beads with those prepared using beads having an iodine content of 155mg I/ml. The figure is also representative particles at 33mg/ml iodine.
***Figure 4*** illustrates results of a saline drop test of representative emulsion preparations. low iodine particles had a 33mg/ml iodine content and high iodine particles had a 155mg/ml iodine content.
***Figure 5*** is a diagrammatic representation of the vascular flow model used in examples.

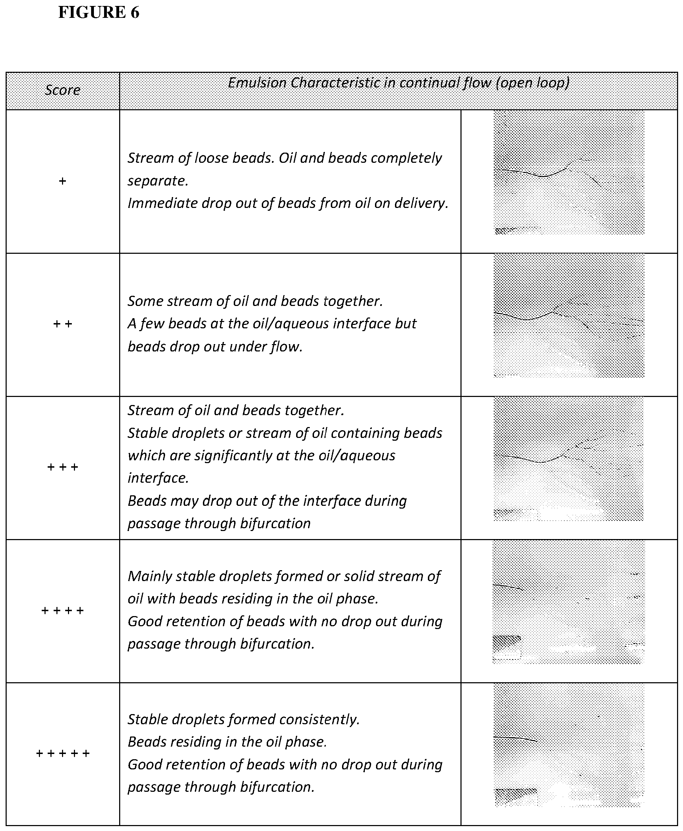

***Figure 6*** illustrates the scoring system for the behaviour of emulsions under flow conditions.
***Figure 7*** shows illustrative high aqueous content and low aqueous content emulsions using high iodine (155mg I/ml) and low iodine (33mg I/ml) microspheres in the flow model.
***Figure 8*** illustrates the effect of re-emulsifying emulsions made with non iodinated and high iodine (147mg I/ml) microspheres after partial separation of theemulsion.
***Figure 9*** illustrates the elution profile of doxorubicin from emulsions of the invention in restricted flow conditions, modelling release of drug from embolic compositions *in vivo.*
***Figure 10*** illustrates the results of atomic force microscopy studies on the microspheres. Figure 9a illustrates the principle of calculating the pull-off (adhesion) force between the silicon tip and the microsphere surface and shows a force-distance curve with cantilever deflection. Figure 9b illustrates the results obtained with an unmodified microsphere, Figure 9c illustrates the results obtained using microspheres with 33mg/ml iodine, figure 9d illustrates the results obtained using microspheres with 155mg/ml iodine.

### Examples:

### Example 1 Preparation of Iodinated Hydrogel Microspheres.

A series of iodinated microspheres were prepared by coupling 2,3,5-triiodobenzaldehyde to preformed PVA-AMPS hydrogel microspheres (DC Bead^{®} Biocompatibles UK Ltd, Farnham; UK). The microspheres were prepared according to Example 1 of WO04071495, high AMPS version up to and including the step of vacuum drying to remove residual solvents. Coupling was then carried out according to Examples 5 and 6 of WO2015/033092, to provide microspheres that comprised triiodobenzyl groups linked via a cyclic acetal to the PVA backbone. Samples were prepared having an iodine content of between 33 and 155 mg/ml packed bead volume and a radiopacity of between 1020 and 6769 HU (measured according to Example 12 of WO2015/033092). Control, non iodinated beads were prepared according to Example 1 of WO04071495, high AMPS version and the process was continued to the end of the dying step. Microspheres were sieved to provide a size range of 70-150um.

This iodine conjugation not only has the effect of increasing the radiopacity of the beads due to the presence of radio-dense iodine atoms, but also introduces hydrophobic moieties into the structure that reduce the water content and alter its surface properties.

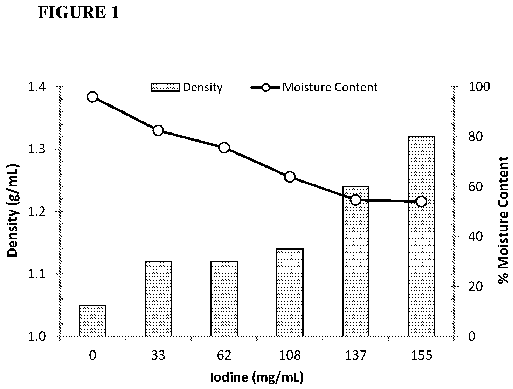

Table 1 illustrates some physicochemical properties of this series of iodine-modified beads. Figure 1 illustrates bead density and moisture content for beads of varying iodine content.

**Table 1: Physicochemical properties of iodinated embolisation microspheres**

| **Characterisation** | **Iodine concentration (mg iodine per mL beads)*** | | | | |
|---|---|---|---|---|---|
| | 33 | 62 | 108 | 137 | 155 |
| **Solid Mass (mg per mL beads)** | 99 | 190 | 288 | 363 | 384 |
| **% Water Content** | 82 | 76 | 64 | 55 | 54 |
| **Radiopacity (HU)** | 1020 | 2120 | 3902 | 5395 | 6769 |

| | | | | | |
|---|---|---|---|---|---|
| **Iodine content is determined per ml, packed volume, of fully hydrated microspheres in normal saline. Once lyophilised, the iodinated microspheres do not return to exactly the same volume on full rehydration, so all measurements of microsphere volume referred to herein refer to the iodine content per ml of fully hydrated microspheres before they have undergone lyophilisation.* | | | | | |

### Example 2: Preparation of particle-oil emulsions

A series of emulsions preparations were made using either non-iodinated or iodinated microspheres (155mg/ml iodine). Individual vials containing 2ml of fully hydrated microspheres (packed volume - measured by measuring cylinder) prepared according to Example 1, were lyophilised and stored dried under vacuum until used. Lipiodol^{®} Ultrafluide (10mL) was added to a vial of dry microspheres through the vial seal without breaking vacuum, via syringe needle, and mixed well for 5 minutes to ensure uptake of the oil into the microspheres. The microspheres were then transferred into a 20mL polypropylene syringe (Becton Dickson). Two millilitres of an aqueous doxorubicin solution (25mg/mL) was aspirated into a 10mL polypropylene syringe (Becton Dickson). Additional water for injection or Omnipaque^{®} 350 contrast agent (GE Healthcare) was added to the doxorubicin solution as required, to obtain the required ratio of oil and aqueous phase when mixed together with the microspheres.

The syringe containing the doxorubicin mixture was attached to the 20mL syringe containing the microspheres and Lipiodol, using a polyamide 3 way stop-cock (Discofix^{®} B.Braun). Initial mixing of the 2 syringe contents was performed by slowly adding the aqueous drug solution to the Lipiodol and microspheres and mixing in order to generate droplets of aqueous drug in the oil phase and to form a homogenous mixture.

The contents were then mixed rapidly between syringes (20 times). This was repeated every 5 minutes for 30 minutes to ensure partition of the drug into and suspension of the microspheres in the emulsion.

The emulsion formulations used are given in Table 3.

### Example 3: Evaluation of Particle-Oil Emulsions

### 3a. Stability:

The prepared particle-oil emulsions were transferred into a 10mL polypropylene syringe (Becton Dickson) and placed in an upright orientation i.e. the length of the syringe in a vertical position. The separation of the oil and the aqueous phase, and the sedimentation of the microspheres in the emulsion were visually monitored. The emulsion stability time was determined as the time taken for separation of the oil and aqueous phases reaching 10% of the initial emulsion volume. In formulations showing settling of microspheres, the time taken for the sedimentation of microspheres to reach approximately 10% of the initial emulsion volume was also noted.

Emulsions with various ratios of oil and water were evaluated for their stability using the most hydrophobic microsphere (155mg/mL iodine) and the hydrophilic microsphere (no iodine). Table 3 reports the comparative stability of emulsions assessed according to the criteria laid out in Table 2.

**Table 2: Assessment Rating Criteria for Emulsion stability**

| Rating | Emulsion Stability (minutes) | Microsphere Sedimentation (minutes) |
|---|---|---|
| + | < 1 | < 1 |
| + + | 2 - 3 | 2 - 3 |
| + + + | 3 - 5 | 3 - 5 |
| + + + + | 5 - 10 | 5 - 10 |
| + + + + + | > 10 | > 10 |

The comparative stability of emulsions containing the hydrophobic microspheres in oil-water ratios ranging from 1:1 to 5:1 was much better than that observed in emulsions with the hydrophilic microspheres (ie no iodine), which were typically only stable for between 2-3 minutes.

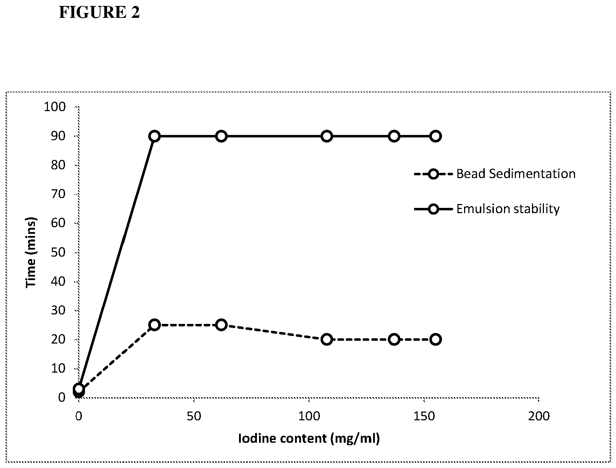

Emulsion stability and bead sedimentation in relation to bead iodine content for a representative emulsion with an oil to aqueous ratio of 2:1 (66.7% Lipiodol, 33.3%aq of which 20% was contrast) are illustrated in Figure 2.

**Table 3: Emulsion Stability Assessment**

| | *Approximate Oil : Water ratio* | *Component Composition* (%) | | | *Emulsion Stability Rating with Microspheres:* | |
|---|---|---|---|---|---|---|
| | | *Lipiodol* | *Aqueous* | | | |
| | | | *Total* | *Contrast Agent* | *Hydrophilic* | *Hydrophobic (155mg*/*mL iodinated)* |
| 1 | 1 : 1 | 52.6 | 47.4 | 15.8 | Not tested | + + + + + |
| 2 | 1.5 : 1 | 58.8 | 41.2 | 17.7 | Not tested | + + + + + |
| 3 | 1.5 : 1 | 58.8 | 41.2 | 29.4 | + + + | + + + + + |
| 4 | 1.5 : 1 | 58.8 | 41.2 | 38.2 | Not tested | + + + + + |
| 5 | 1.5 : 1 | 62.5 | 37.5 | 0.0 | Not tested | + + + + + |
| 6 | 2 : 1 | 66.7 | 33.3 | 0.0 | Not tested | + + + + + |
| 7 | 2 : 1 | 66.7 | 33.3 | 20.0 | + + | + + + + + |
| 8 | 2.5 : 1 | 71.4 | 28.6 | 3.6 | + + | + + + + + |
| 9 | 5 : 1 | 83.3 | 16.7 | 12.5 | Not tested | + + + + + |
| 10 | 5 : 1 | 83.3 | 16.7 | 0.0 | Not tested | + + + + + |
| 11 | 2 : 1 | 66.7 | 33.3 | 25.0 | + + | Not tested |
| 12 | 2.2 : 1 | 68.9 | 31.1 | 17.2 | + + | Not tested |
| 13 | 2.5 : 1 | 71.4 | 28.6 | 14.3 | + | Not tested |
| 14 | 2.5 : 1 | 71.4 | 28.6 | 21.4 | + | Not tested |
| 15 | 3 : 1 | 74.1 | 25.9 | 11.1 | + | Not tested |

### 3b. Appearance:

A drop of emulsion containing either non iodinated or iodinated microspheres (155 or 33 mg/ml iodine) was placed onto a petri dish and immediately covered with a glass cover slip. Optical micrographs (x4 or x10 magnification) were obtained using a BX50 microscope, Colorview III Camera and Stream Essential imaging software (Olympus). In order to differentiate between the oil and aqueous phase, an aqueous solution of Reactive Blue 4 dye in water (50mg/mL) was added dropwise to the emulsion on the petri dish. The aqueous phase of the emulsion could be identified by the migration of the blue dye solution towards it under the microscope. The type of emulsion (oil-in-water or a water-in-oil) and the location of the microspheres, in relation to the oil or aqueous phases, were noted.

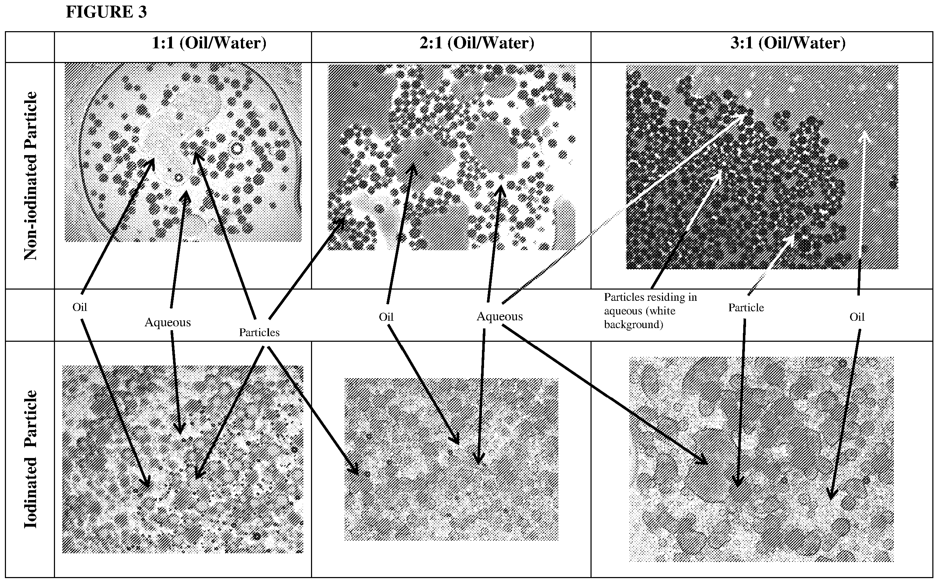

Figure 3 illustrates particle-oil emulsions. The iodinated microspheres have a preference to reside in the oil phase despite their relatively high water content, and accumulate at the liquid droplet interface, hence stabilising the droplets from coalescence. Even at the 1 : 1 ratio of oil to aqueous, where the continuous phase of the emulsion appears to be the aqueous, the iodinated microspheres are still predominantly populated around the oil droplets and reside at the interface between the water and oil. The non-iodinated microspheres are more hydrophilic in comparison and are observed to reside in the aqueous phase, even in the emulsions with a high oil composition (i.e. 2 : 1 and 3 : 1 oil-water ratio).

### 3c. Emulsion stability (cohesiveness) in static flow conditions (Saline droplet test)

A sample of emulsion was delivered below the surface of a 0.9% saline solution through an 18 gauge blunt fill needle (Becton Dickson) at room temperature. The appearance and behaviour of the emulsion, was observed and rated according to the characteristics defined in Table 4. The emulsions appearing as spherical droplets containing the microspheres without fragmenting or disintegrating were considered to have the better flow behaviour and emulsion stability.

**Table 4: Assessment Rating Criteria for Flow Characteristics in Static Conditions**

| **Rating** | **Emulsion Characteristic in static flow** |
|---|---|
| + | Loose beads separated from oil and residing in the aqueous phase i.e. no association of beads with oil. |
| + + | Stream of oil with some beads associated with oil. Beads mainly residing in the aqueous phase (saline). |
| + + + | Stream of oil where beads are associated with the oil. Forms droplets with beads significantly at the oil/aqueous interface. Beads may aggregate towards the aqueous. |
| + + + + | Stable oil droplets containing beads. Some streams of oil and beads combined - beads within the stream are visually bound together by the oil. |
| + + + + + | Stable oil droplets in which the beads reside are formed consistently. Oil droplets hold together. |

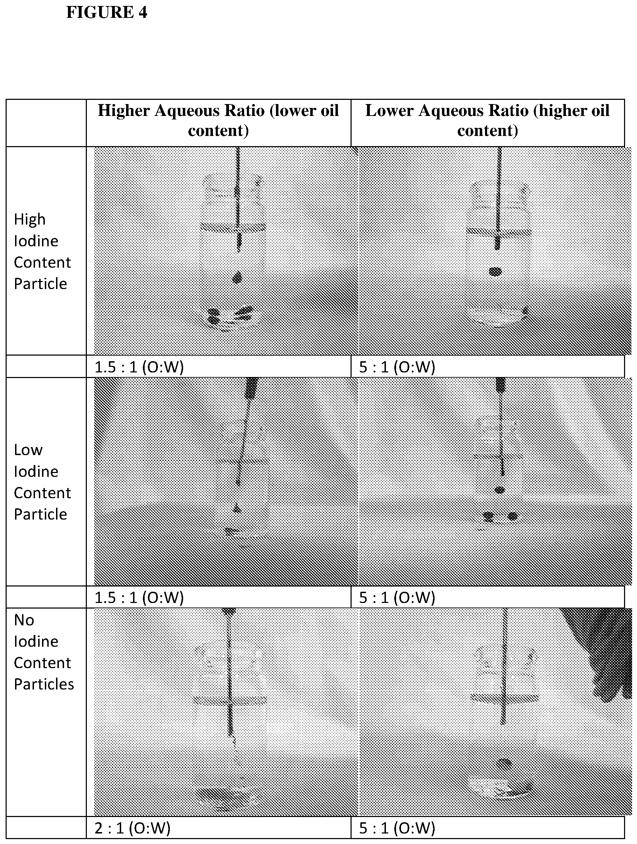

Figure 4 illustrates the flow characteristics observed in representative particle-oil emulsions having oil to water ratios ranging from approximately 1.5 : 1 to 5 : 1. The more hydrophobic microspheres containing iodine resulted in droplets that were more spherical and had better flow characteristics than the comparatively hydrophilic microspheres containing no iodine. Increasing aqueous content in the particle-oil emulsions resulted in droplets that were less spherical. This was more apparent in microspheres containing no iodine. Table 5 illustrates the results

**Table 5: Behaviour of emulsions containing hydrophobic microspheres (33 and 155mg/mL) and hydrophilic microspheres under static flow**

| | *Approximate Oil : Water ratio* | *Component Composition* (%) | | | *Emulsion Flow Rating with Microspheres:* | |
|---|---|---|---|---|---|---|
| | | *Lipiodol* | *Aqueous* | | | |
| | | | *Total* | *Contrast Agent* | *Hydrophilic* | *Hydrophobic * (iodinated)* |
| 1 | 1 : 1 | 52.6 | 47.4 | 15.8 | Not tested | + + + |
| 2 | 1.5 : 1 | 58.8 | 41.2 | 17.7 | Not tested | + + + |
| 3 | 1.5 : 1 | 58.8 | 41.2 | 29.4 | + | + + + - |
| 4 | 1.5 : 1 | 58.8 | 41.2 | 38.2 | Not tested | + + + + |
| 5 | 1.5 : 1 | 62.5 | 37.5 | 0.0 | Not tested | + + + + |
| 6 | 2 : 1 | 66.7 | 33.3 | 0.0 | Not tested | + + + + + |
| 7 | 2 : 1 | 66.7 | 33.3 | 20.0 | + + | + + + + + |
| 8 | 2.5 : 1 | 71.4 | 28.6 | 3.6 | + + | + + + + + |
| 9 | 5 : 1 | 83.3 | 16.7 | 12.5 | Not tested | + + + + + |
| 10 | 5 : 1 | 83.3 | 16.7 | 0.0 | Not tested | + + + + + |
| 11 | 2 : 1 | 66.7 | 33.3 | 25.0 | + + | Not tested |
| 12 | 2.2 : 1 | 68.9 | 31.1 | 17.2 | + + + | Not tested |
| 13 | 2.5 : 1 | 71.4 | 28.6 | 14.3 | + + + | Not tested |
| 14 | 2.5 : 1 | 71.4 | 28.6 | 21.4 | + + + | Not tested |
| 15 | 3 : 1 | 74.1 | 25.9 | 11.1 | + + + | Not tested |

| | | | | | | |
|---|---|---|---|---|---|---|
| **Microspheres having 33mg I*/*ml and 155 mg I*/*ml behave the same.* | | | | | | |

Emulsions containing iodinated microspheres at both 33mg/mL and 155mg/mL levels, formed stable oil droplets containing the microspheres, in compositions where the oil : water ratio was at least 2 : 1. At lower oil-water ratios, the emulsion was still able to form stable oil droplets containing the particles.

In comparison, the microspheres containing no iodine showed flow behaviour that was poor in comparison to the iodinated samples. Additional formulations with these microspheres were tested, with oil-water ratios ranging from 2:1 to 3:1 and varying contrast agent compositions. These did not improve the emulsion flow behaviour such that it was comparable to that observed with hydrophobically modified microspheres.

### 3d. Behaviour under continual flow

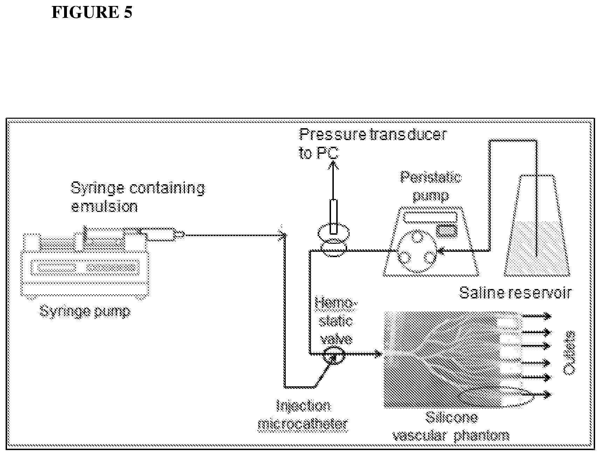

An *in vitro* vascular flow simulator (Figure 5) was used to profile the physical stability of emulsions under continual flow conditions. The particle-oil emulsions tested under static flow conditions (in "c." above) were tested under non-restricted continual flow conditions.

The emulsions were transferred into a 3mL polypropylene syringe (Becton Dickson) attached to microcatheter (Progreat 2.4Fr, Terumo) for administration into a silicone vascular flow simulator model (Elastrat Sarl, Switzerland) set-up as an open loop system.

The distal end of the microcatheter was located proximally in the vascular channels and a 0.9% saline solution at ambient temperature was pumped through the flow model at a pulsatile rate of 60mL/minute, by a peristaltic pump. The particle-oil emulsion was administered manually through the microcatheter at an injection rate of approximately 0.5mL/minute. The appearance of the emulsion delivered from the microcatheter and into the vascular flow model and delivery channels was observed for its flow characteristics (Table 5) and was graded for the ability to form discreet oil droplets and maintain the droplet integrity during its flow through the in vitro vascular network. The emulsions appearing as discreet spherical oil droplets containing the microspheres were considered to have the best flow behaviour and stability i.e. high scoring, whereas streaming beads (not within the oil droplet) were defined as being less stable and low scoring.

The minimum requirement for acceptable stability being that the emulsion had a rating of (+ + +) i.e. appeared as a stream of oil and beads together or as stable droplets or a stream of oil containing beads which are significantly at the oil/aqueous interface.

**Table 6: Assessment Rating Criteria for Flow Characteristics in Continuous flow**

| **Score** | **Emulsion Characteristic in continual flow (open loop)** |
|---|---|
| + | *Stream of loose beads. Oil and beads completely separate.* |
| | *Immediate drop out of beads, from oil on delivery.* |
| ++ | *Some stream of oil and beads together.* |
| | *A few beads at the oil*/*aqueous interface but beads drop out under flow.* |
| + + + | *Stream of oil and beads together.* |
| | *Stable droplets or stream of oil containing beads which are significantly at the oil*/*aqueous interface.* |
| | *Beads may drop out of the interface during passage through bifurcation* |
| + + + + | *Mainly stable droplets formed or solid stream of oil with beads residing in the oil phase.* |
| | *Good retention of beads with no drop out during passage through bifurcation.* |
| + + + + + | *Stable droplets formed consistently. Beads residing in the oil phase. Good retention of beads with no drop out during passage through bifurcation.* |

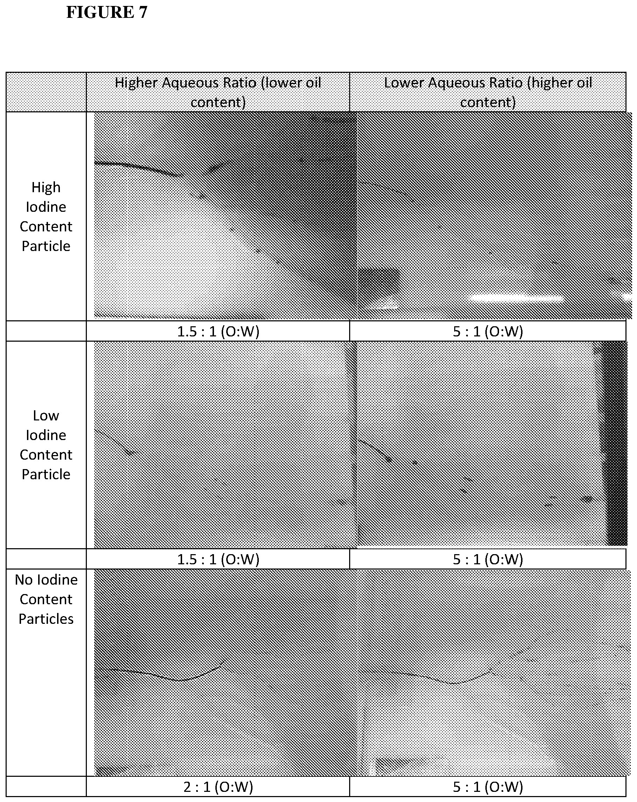

Figure 7 illustrates example emulsions in flow conditions and shows that under continual flow, the emulsions prepared with the microspheres containing iodine consistently produce stable droplets in which the microspheres reside, with complete retention as the droplets pass through the bifurcations of the vascular model.

Increasing aqueous content in the particle-oil emulsions has a direct impact on their flow characteristics with the non iodine microspheres not able to form stable droplets of oil, containing the microspheres, even when comprising a high oil content. In comparison, the hydrophilic microspheres did not form robust droplets containing beads. In emulsion compositions with high oil content, oil droplets did form but the microspheres tended to reside at the interface of the oil and had a tendency to be displaced and fall out under continual flow.

Table 7 reports the assessment of flow characteristics according to Table 5.

**Table 7 Behaviour of emulsions containing hydrophobic microspheres (33 and 155mg/mL) under continual flow**

| | *Approximate Oil : Water ratio* | *Component Composition* (%) | | | *Emulsion Flow Rating with Microspheres:* | |
|---|---|---|---|---|---|---|
| | | *Lipiodol* | *Aqueous* | | | |
| | | | *Total* | *Contrast Agent* | *Hydrophilic* | *Hydrophobic (iodinated)* |
| 1 | 1 : 1 | 52.6 | 47.4 | 15.8 | Not tested | + + + |
| 2 | 1.5 : 1 | 58.8 | 41.2 | 17.7 | Not tested | + + + + |
| 3 | 1.5 : 1 | 58.8 | 41.2 | 29.4 | + | + + + + + |
| 4 | 1.5 : 1 | 58.8 | 41.2 | 38.2 | Not tested | + + + + + |
| 5 | 1.5 : 1 | 62.5 | 37.5 | 0.0 | Not tested | + + + + + |
| 6 | 2 : 1 | 66.7 | 33.3 | 0.0 | Not tested | + + + + + |
| 7 | 2 : 1 | 66.7 | 33.3 | 20.0 | + + | + + + + + |
| 8 | 2.5 : 1 | 71.4 | 28.6 | 3.6 | + + + | + + + + + |
| 9 | 5 : 1 | 83.3 | 16.7 | 12.5 | Not tested | + + + + + |
| 10 | 5 : 1 | 83.3 | 16.7 | 0.0 | Not tested | + + + + + |
| 11 | 2 : 1 | 66.7 | 33.3 | 25.0 | + + | Not tested |
| 12 | 2.2 : 1 | 68.9 | 31.1 | 17.2 | + + + | Not tested |
| 13 | 2.5 : 1 | 71.4 | 28.6 | 14.3 | + + + | Not tested |
| 14 | 2.5 : 1 | 71.4 | 28.6 | 21.4 | + + + | Not tested |
| 15 | 3 : 1 | 74.1 | 25.9 | 11.1 | + + + | Not tested |

These findings are supported *in vivo* following fluoroscopic guided delivery in healthy swine and evaluation of flow properties. Iodinated microspheres (XXmg I/ml - 70-150um) flowed as discreet packets of emulsion as per the *in vitro* conclusions, whilst cTACE Lipiodol emulsions and emulsions prepared with unmodified microspheres presented loose "whispy" flow properties indicative of poor flow stability.

### 3e. Effect of aqueous phase density on Particle-oil emulsions

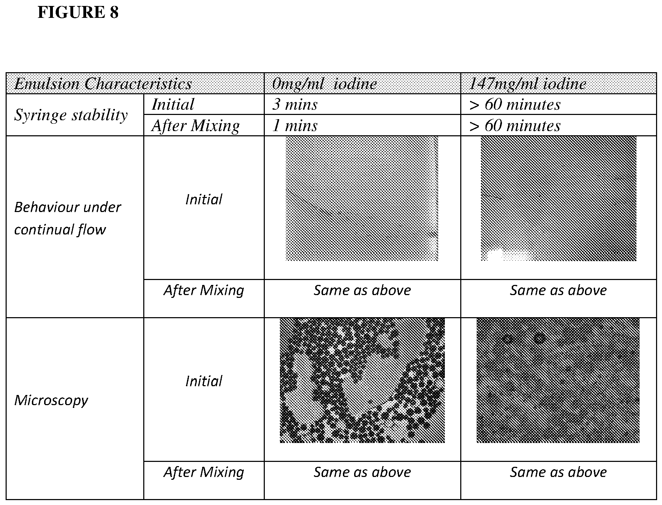

The behaviour of the particle-oil emulsion when the aqueous phase is water (with a much lower specific gravity than that of the contrast agent) is comparable to emulsions in which the aqueous phase comprises contrast agent. Figure 8 shows representative images of the flow characteristics observed in particle-oil emulsions which were prepared using either water or contrast agent, in an oil-water ratio of 2 : 1 (Table 8).

**Table 8: Composition of Emulsion**

| *% Lipiodol* | *% Aqueous* | *% Contrast Agent component (in Aqueous)* |
|---|---|---|
| *66.7* | *33.3* | *20.0* |
| *66.7* | *33.3* | *0* |

The homogeneity of the emulsion droplet in the static flow test (saline drop) was slightly less in the emulsion prepared with water only, however, under continual flow it was comparable to the emulsion prepared with contrast agent.

### 3f. Emulsion Characteristics on Re-mixing

The particle-oil emulsions were evaluated for their flow characteristics and their appearance after initial preparation and following phase separation and subsequent re-mixing. Particle-oil emulsions containing the more hydrophilic microspheres, having no bound iodine, do not form stable and robust emulsion under flow or in the syringe. Once phase separation has occurred (3 minutes), re-mixing of the emulsion exacerbated the separation and overall syringe stability was reduced further to 1 minute.

In contrast, the iodine containing microspheres formed an emulsion which was stable for over 60 minutes and once re-mixed was still stable in the syringe and able to form stable droplets under continual flow. On phase separation and coalescence of the oil and aqueous droplets, the hydrophobic microspheres are capable of stabilising the emulsion after re-mixing, and demonstrating their original characteristics (see Figure 8).

### 3g. In vitro drug elution behaviour of emulsions in a vascular flow simulator with restricted flow

The drug release from the particle oil emulsions was profiled under continual flow conditions with a partially restricted flow rate on sample delivery. The particulate-oil emulsions and in vitro system were prepared as in Section 3d but with the *in vitro* system set-up as a semi closed-loop system as follows.

A single sample delivery channel was restricted using a 27µm microporous mesh (woven polyamide) on its outlet port to simulate the effect of flow confinement in micro-channels once the emulsion was administered. The other outlets on the silicone vascular phantom, which were not used for sample delivery, were left unrestricted, with their outlets recirculating the elution media (0.9% saline solution) back into the saline reservoir (heated to 37°C). The microcatheter was placed distally into the single sample delivery channel and the saline solution was pumped through the restricted sample delivery channel at a flow rate of approximately 80mL/minute.

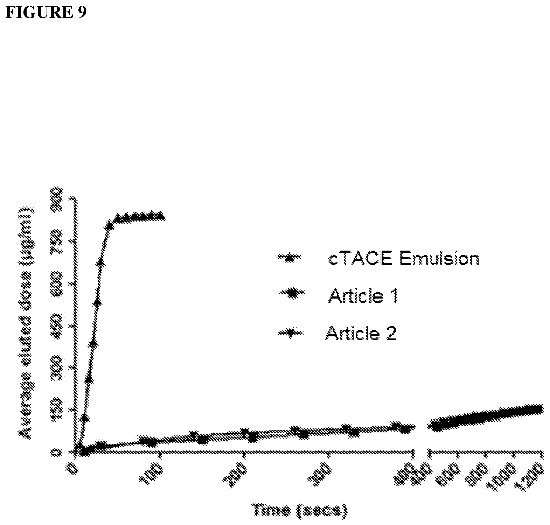

The particle-oil emulsion prepared as above were administered through the microcatheter using a syringe auto-injector (PHD Ultra; Harvard Apparatus) at an injection rate of 0.5mL/minute. The emulsions were delivered until the flow rate at the exit port of the delivery channel was observed to have significantly reduced. During the delivery of the emulsion, the saline from the sample delivery channel was captured and analysed by UV/Vis spectrophotometry (Cary 50 Bio, Varian) at a wavelength of 483nm to determine the amount of doxorubicin eluted. The cumulative drug eluted over a 20min time period, was normalised by determining it as a percentage of the theoretical dose administered. Table 9 give the details of the emulsions for which data is shown in Figure 9.

**Table 9: Composition of emulsions tested in Example 3g**

| | Iodine | O : W Ratio | Lipiodol % | Total Aqueous % | of which Contrast *Agent* is | details |
|---|---|---|---|---|---|---|
| Article 1 Low Aqueous | 155 | 5 : 1 | 83.3 | 16.7 | 0.0 | 10mL oil + 2mL dox in water |
| Article 2 Low Aqueous | 0 | 5 : 1 | 83.3 | 16.7 | 0.0 | 10mL oil + 2mL dox in water |
| cTACE | - | 5 : 1 | 83.3 | 16.7 | 0.0 | 10mL oil + 2mL dox in water |

### 4a. Measurement of microsphere surface properties

Relative hydrophilicity/hydrophobicity of the microsphere surfaces was measured by use of Contact Atomic Force Spectroscopy using force-distance-amplitude protocols.

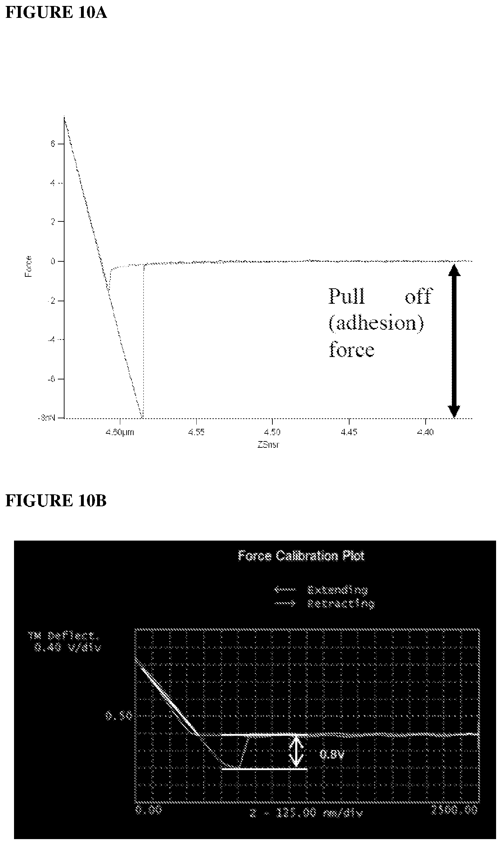

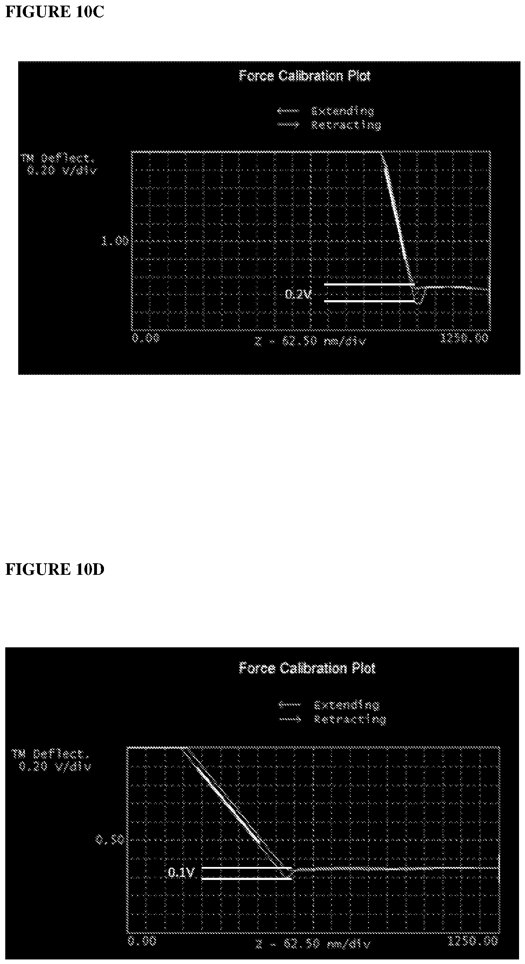

Initial qualitative assessments were performed on a Dimension 3000 atomic force microscope (AFM) (Digital Instruments) and data collected using the NanoScope IIIA software (Digital Instruments). The force distance curve was obtained by measuring the deflection signal (voltage) from the cantilever as the probe approached the surface from approximately 750-1800nm above the surface at a constant scan rate of 1Hz. The cantilever deflection was observed as the difference in signal voltage at the points when the tip is retracting from the microsphere surface and when the tip is free from the surface (Figure 10). Using the silicon AFM probe (PointProbe NCH-W; Nanosensors), it was expected that the adhesion and cantilever deflection would be highest in microspheres having a more hydrophilic surface property.

The microsphere containing no iodine has the highest cantilever deflection signal of 0.8V and the lowest signal of 0.1V was observed in the microsphere with the highest amount of iodine (Table 10), thus indicating comparative hydrophobicity increases with increasing iodine in the microspheres.

**Table 10 AFM Cantilever Deflection Signals in Microspheres of Differing Iodine Content**

| Iodine content (mg/mL) | Cantilever Deflection (V) |
|---|---|
| 0 | 0.8 |
| 33 | 0.2 |
| 155 | 0.1 |

### 4b alternate AFM measurement

Further verification of the adhesion force was performed using an Asylum Research Cypher AFM instrument (Oxford Instruments) and a CONTSCR-10 cantilever (silicon probe) (Nanoworld). A force map was generated for each microsphere within an overall scan size area of 4µm by 4µm. The force map comprised a total of 256 individual force distance curves (within the scan area) which were taken 250µm apart from each other (i.e. in a regular array of 16 x 16 measurements).

The force-distance curves were obtained by measuring the deflection of the AFM cantilever (holding an uncoated silicon probe) as it approached and retracted from the microsphere surface. Each curve was taken at a constant scan rate of 1Hz, and comprised of 2000 points during the approach and retract cycle. The tip of the probe started 300nm above the microsphere and was pressed 20nm into the microsphere surface before being retracted. Depending on the curvature of the microsphere surface, the z height (distance of the AFM probe tip above the surface of the microsphere) over the area scanned was approximately 300 - 800nm. The cantilever deflection was measured as a signal voltage and was calculated to give an adhesion force using the spring constant of the cantilever.

These assessments to determine the adhesion force on the hydrophobic microspheres (Table 11) show that the hydrophobic microsphere with the lower iodine concentration has the largest pull-off force and therefore the greater adhesion with the silicon AFM probe. Since the probe is considered to be hydrophilic, the lower iodine sample is determined to be less hydrophobic than the higher iodine sample.

**Table 11 AFM Adhesion Forces in Microspheres Containing Iodine**

| Sample | Average pull-off force (nN) | |
|---|---|---|
| | Replicate 1 | Replicate 2 |
| Low Iodine Microsphere (33mg/mL ) | 11.6 ±8.8 | 8.6 ± 4.7 |
| High Iodine Microsphere (155mg/mL ) | 4.1 ± 1.4 | 4.4 ± 1.5 |

## Claims

1. An emulsion composition comprising a continuous phase, a discontinuous phase and a plurality of particles, the discontinuous phase being aqueous and the continuous phase comprising an oil;
wherein the particles comprise a hydrophilic polymer to which iodine is covalently bound.

2. A composition according to claim 1 wherein the iodine is bound to an aromatic group, which aromatic group is covalently bound to the hydrophilic polymer.

3. A composition according to any preceding claim wherein particles comprise a hydrophilic polymer to which iodine is covalently bound, and wherein the iodine is present in the particles at a level of at least 30 mg I/ml of packed volume, in particular 60 mg iodine per ml PV.

4. A composition according to any preceding claim wherein the particles comprise a hydrophilic hydrogel polymer.

5. A composition according to any preceding claim wherein the hydrophilic polymer carries an overall charge at a pH between 6 and 8.

6. A composition according to claim 5, wherein the hydrophilic polymer carries an anionic charge at a pH between 6 and 8.

7. A composition according to any preceding claim wherein the volume of oil phase exceeds the volume of aqueous phase, in particular where the ratio of oil to aqueous phase is greater than 1.1: 1 v/v.

8. A composition according to any preceding claim, which is stable for at least 10 minutes at between 18 and 22°C.

9. A composition according to any preceding claim wherein the hydrophilic polymer comprises polyvinyl alcohol.

10. A composition according to any preceding claim wherein the aqueous phase comprises a contrast agent.

11. A composition according to any preceding claim wherein the particles comprise a pharmaceutical active ingredient.

12. A composition according to any preceding claim where in the oil is a composition of iodised ethyl-esters of the fatty acids of poppy seed oil.

13. A process for preparing an emulsion according to any preceding claim comprising:
a. providing a continuous phase comprising an oil;
b. providing an aqueous phase
c. providing a plurality of particles comprising a hydrophilic polymer to which
iodine is covalently bound; and
combining them to provide an emulsion.

14. A pharmaceutical active for use in the treatment of a tumour by embolotherapy, wherein the pharmaceutical active is delivered in an emulsion composition according to any of claims 1 to 12.

15. A kit for preparing an emulsion composition according to any one of claims 1 to 12, comprising an oil and a plurality of particles, the particles comprising a hydrophilic polymer to which iodine is covalently bound.

## Patentansprüche

1. Emulsionszusammensetzung, umfassend eine kontinuierliche Phase, eine diskontinuierliche Phase und eine Mehrzahl von Teilchen, wobei die diskontinuierliche Phase wässrig ist und die kontinuierliche Phase ein Öl umfasst; wobei die Teilchen ein hydrophiles Polymer umfassen, an das Iod kovalent gebunden ist.

2. Zusammensetzung nach Anspruch 1 wobei, das Iod an eine aromatische Gruppe gebunden ist, wobei die aromatische Gruppe kovalent an das hydrophile Polymer gebunden ist.

3. Zusammensetzung nach einem der vorstehenden Ansprüche, wobei die Teilchen ein hydrophiles Polymer umfassen, an das Iod kovalent gebunden ist, und wobei das Iod in den Teilchen in einer Menge von mindestens 30 mg I/ml Packvolumen, insbesondere 60 mg Iod pro ml PV, enthalten ist.

4. Zusammensetzung nach einem der vorstehenden Ansprüche, wobei die Teilchen ein hydrophiles Hydrogelpolymer umfassen.

5. Zusammensetzung nach einem der vorstehenden Ansprüche, wobei das hydrophile Polymer bei einem pH-Wert zwischen 6 und 8 eine Gesamtladung trägt.

6. Zusammensetzung nach Anspruch 5, wobei das hydrophile Polymer bei einem pH-Wert zwischen 6 und 8 eine anionische Ladung trägt.

7. Zusammensetzung nach einem der vorstehenden Ansprüche, wobei das Volumen der Ölphase das Volumen der wässrigen Phase übersteigt, wobei das Verhältnis von Ölzu wässriger Phase insbesondere größer als 1,1 : 1 Vol./Vol. ist.

8. Zusammensetzung nach einem der vorstehenden Ansprüche, die zwischen 18 und 22 °C mindestens 10 Minuten lang stabil ist.

9. Zusammensetzung nach einem der vorstehenden Ansprüche, wobei das hydrophile Polymer Polyvinylalkohol umfasst.

10. Zusammensetzung nach einem der vorstehenden Ansprüche, wobei die wässrige Phase ein Kontrastmittel umfasst.

11. Zusammensetzung nach einem der vorstehenden Ansprüche, wobei die Teilchen einen pharmazeutischen Wirkstoff umfassen.

12. Zusammensetzung nach einem der vorstehenden Ansprüche, wobei in dem Öl eine Zusammensetzung von iodierten Ethylestern der Fettsäuren von Mohnöl ist.

13. Verfahren zum Herstellen einer Emulsion nach einem der vorstehenden Ansprüche, umfassend:
a. Bereitstellen einer kontinuierlichen Phase, die ein Öl umfasst;
b. Bereitstellen einer wässrigen Phase;
c. Bereitstellen einer Mehrzahl von Teilchen, die ein hydrophiles Polymer umfassen, an das Iod kovalent gebunden ist; und Zusammenführen derselben, um eine Emulsion bereitzustellen.

14. Pharmazeutischer Wirkstoff zur Verwendung bei der Behandlung eines Tumors mittels Embolotherapie, wobei der pharmazeutische Wirkstoff in einer Emulsionszusammensetzung nach einem der Ansprüche 1 bis 12 abgegeben wird.

15. Kit zum Herstellen einer Emulsionszusammensetzung nach einem der Ansprüche 1 bis 12, umfassend ein Öl und eine Mehrzahl von Teilchen, wobei die Teilchen ein hydrophiles Polymer umfassen, an das Iod kovalent gebunden ist.

## Revendications

1. Composition d'émulsion comprenant une phase continue, une phase discontinue et une pluralité de particules, la phase discontinue étant aqueuse et la phase continue comprenant une huile ;
les particules comprenant un polymère hydrophile auquel de l'iode est lié de manière covalente.

2. Composition selon la revendication 1, l'iode étant lié à un groupe aromatique, lequel groupe aromatique étant lié de manière covalente au polymère hydrophile.

3. Composition selon une quelconque revendication précédente, des particules comprenant un polymère hydrophile auquel de l'iode est lié de manière covalente, et l'iode étant présent dans les particules à un taux d'au moins 30 mg de I/ml de volume tassé, en particulier 60 mg d'iode par ml de PV.

4. Composition selon une quelconque revendication précédente, les particules comprenant un polymère d'hydrogel hydrophile.

5. Composition selon une quelconque revendication précédente, le polymère hydrophile portant une charge globale à un pH entre 6 et 8.

6. Composition selon la revendication 5, le polymère hydrophile portant une charge anionique à un pH entre 6 et 8.

7. Composition selon une quelconque revendication précédente, le volume de phase huileuse dépassant le volume de phase aqueuse, en particulier le rapport de phase huileuse sur aqueuse étant supérieur à 1,1:1 v/v.

8. Composition selon une quelconque revendication précédente, qui est stable pendant au moins 10 minutes à une température comprise entre 18 et 22 °C.

9. Composition selon une quelconque revendication précédente, le polymère hydrophile comprenant un poly(alcool vinylique).

10. Composition selon une quelconque revendication précédente, la phase aqueuse comprenant un agent de contraste.

11. Composition selon une quelconque revendication précédente, les particules comprenant un ingrédient actif pharmaceutique.

12. Composition selon une quelconque revendication précédente, l'huile étant une composition d'esters d'éthyle iodisés des acides gras d'huile de graines de pavot.

13. Procédé pour la préparation d'une émulsion selon une quelconque revendication précédente comprenant :
a. la fourniture d'une phase continue comprenant une huile ;
b. la fourniture d'une phase aqueuse
c. la fourniture d'une pluralité de particules comprenant un polymère hydrophile auquel de l'iode est lié de manière covalente ; et
la combinaison de celles-ci pour fournir une émulsion.

14. Agent actif pharmaceutique pour une utilisation dans le traitement d'une tumeur par embolothérapie, l'agent actif pharmaceutique étant délivré dans une composition d'émulsion selon l'une quelconque des revendications 1 à 12.

15. Kit pour la préparation d'une composition d'émulsion selon l'une quelconque des revendications 1 à 12, comprenant une huile et une pluralité de particules, les particules comprenant un polymère hydrophile auquel de l'iode est lié de manière covalente.
